# EUROPEAN PATENT APPLICATION

(11) **EP 4 610 991 A1**
(43) Date of publication of application: **03.09.2025**
(21) Application number: 25160565.5
(22) Date of filing: 27.02.2025
(51) Int. Cl.: G16H 10/40, G16H 40/20

(54) **A COMPUTER-IMPLEMENTED METHOD FOR PREDICTING FUTURE OPERATING CONDITIONS OF A HEALTHCARE LABORATORY**

(30) Priority: 29.02.2024 EP 24160703
(71) Applicant: F. Hoffmann-La Roche AG, 4070 Basel (CH); Roche Diagnostics International AG, 6343 Rotkreuz (CH); Roche Diagnostics GmbH, 68305 Mannheim (DE)
(72) Inventor: EZZI, Afshin, 6343 Rotkreuz ZG (CH); GUTMANN, Oliver, 6343 Rotkreuz ZG (CH); JAGDHUBER, Rudolf, 82377 Penzberg (DE); KING, Michael, 82377 Penzberg (DE); MENARD, Jeffrey Drew, 6343 Rotkreuz ZG (DE); SCHWAB, Wolfgang Leonhard, 4070 Basel BS (CH)
(74) Representative: Mewburn Ellis LLP

(57) **Abstract**

A computer-implemented method for predicting future operating conditions of a healthcare laboratory is provided. The healthcare laboratory contains one or more in-vitro diagnostic laboratory instruments configured to process medical samples. The method comprises: receiving medical sample event data describing events relating to one or more medical samples to be processed by the laboratory, augmenting the medical sample event data with laboratory data; creating a feature set from the augmented medical sample event data, the feature set including time bracketed data derived from the augmented medical sample event data; using the feature set as an input for a trained machine learning model, the machine learning model having been trained on historical feature sets to predict a future medical sample events within a forecast time range; and receiving, from the trained machine learning model, a prediction of future medical sample events for the forecast time range as an output.

## Description

### TECHNICAL FIELD

The present disclosure relates to a computer-implemented method for predicting future operating conditions of a healthcare laboratory.

### BACKGROUND

Modern healthcare laboratories for processing medical samples are increasingly employing automated processes into their operation. Such laboratories typically have the capacity to process thousands of samples per day, and larger automated laboratories even process up to 100,000 samples per day. In such automated systems, the processes are typically monitored by a central computer system, which is responsible for managing the flow of medical samples through the laboratory. The central computer system often also performs quality control and generates test reports. The system may monitor the ordering of medical processing of samples by, for example, a hospital or general medical practitioner, as well monitoring the transportation, processing and return of the medical samples. Such central processing systems are typically able to generate reports on the status of the medical sample processing of the laboratory.

These reports aim to enable a manager of the healthcare laboratory to efficiently allocate laboratory staff and sample processing devices to process the required medical samples. However, conventional systems do not access sufficient data to properly inform these decisions, and provide nothing beyond real-time values relating to medical samples within the system.

The present disclosure was arrived at in light of the above considerations.

### SUMMARY

Accordingly, in a first aspect, embodiments of the present invention provide a computer-implemented method for predicting future operating conditions of a healthcare laboratory, the healthcare laboratory containing one or more in-vitro diagnostic laboratory instruments configured to process medical samples, the method comprising:
receiving medical sample event data describing events relating to one or more medical samples to be processed by the laboratory,
augmenting the medical sample event data with laboratory data;
creating a feature set from the augmented medical sample event data, the feature set including time bracketed data derived from the augmented medical sample event data;
using the feature set as an input for a trained machine learning model, the machine learning model having been trained on historical feature sets to predict a future medical sample events within a forecast time range; and
receiving, from the trained machine learning model, a prediction of future medical sample events for the forecast time range as an output.

In-vitro diagnostic laboratory instruments may be pre-analytical instruments, analytical instruments, post-analytical instruments, or instruments used in the transportation of the medical samples. For example, the healthcare laboratory may be an in-vitro diagnostic laboratory including at least one analytical instrument.

Medical sample event data may comprise, for example, data describing the ordering of a medical sample, and data describing the transit of the medical sample between the laboratory and the sample ordering entity. The medical sample data relating to a specific event may describe, for example, the date and time of the event, as well as optionally containing further event specific metadata. Medical sample event data may be understood as pre-lab data, that is, data relating to the samples before they have been delivered to and therefore processed by the laboratory. It may further describe: the number of samples collected from different healthcare providers; the date and time that a given sample is ordered; data available from one or more pre-laboratory sample tracking systems; and data available from one or more systems related to couriers or transporters of samples outside of the laboratory. The medical sample event data may, for example, be from a pre-laboratory sample tracking system and/or systems related to couriers or transporters of samples. By time bracketed data, it may be meant data where values are binned according to their time value into a corresponding bin. For example, the time bracketed data may include bins with time labels indicating time periods. Corresponding counters may indicate the number of data points which reside within a given category for that time period. In one example, the time bracketed data is ordered by hour periods on a given day, or the time bracketed data may be ordered by day.

In one example, the laboratory data includes dynamic laboratory data and static laboratory data.

The laboratory data may be understood as in-lab data, that is data originating from within and relating to the laboratory. For example, the number of samples arriving in the or each of a plurality of laboratories over time as historical data; when a sample arrived at the laboratory; when different processing steps occurred in relation to the sample (e.g., data created by instruments and collected by middleware); data describing events occurring relative to the sample; data from analytical, pre-analytical, or post-analytical instruments; data from information systems (e.g., a laboratory information system, healthcare information system, and/or middleware); and data available from sample monitoring applications.

The laboratory data may comprise static and dynamic data. Static data can be understood as data which does not vary, or does not significantly vary, with time (for example the average time taken for a sample to get from a clinic to the laboratory). Dynamic data can be understood as data which does vary with time (for example the number of samples to be processed, the amount of reagent available within an analytical instrument, etc.). Static laboratory data therefore is laboratory data, which is not undergoing continuous change and may describe, for example, in-vitro diagnostic laboratory instrument throughput data, in-vitro diagnostic laboratory instrument availability data, staff schedule data, workflow definitions (such as process flows for given tests, staff allocation for a given test, priorities for given tests, and/or instrument settings for given tests) and/or laboratory layout data. Dynamic laboratory data is continuously changing, and may describe, for example, the medical sample event data of medical samples currently being processed (in contrast to samples to be processed by the laboratory), current reagent levels of the laboratory, and/or the current status of each in-vitro diagnostic laboratory instrument. Static medical sample event data may include, for example, an average time taken to transport a sample from a clinic to the laboratory for processing. Dynamic medical sample event data may include, for example, the location of the medical sample. The feature set may be a data set containing features, each feature being a measurable property derived from the augmented medical sample event data. For example, the average number of samples ordered may be used as a feature. A value may be calculated for each feature for each time bracket of the time bracketed data. The dynamic laboratory data may, for example, be from an in-vitro diagnostic laboratory instrument, an information system of the healthcare laboratory (e.g., a laboratory information system or healthcare information system), and/or a middleware (e.g., a system for managing the in-vitro diagnostic laboratory instruments and interfacing with the information system of the healthcare laboratory). The static laboratory data may, for example, be from a database of laboratory data, be from in-vitro diagnostic laboratory instruments, an information system of the healthcare laboratory, and/or a middleware.

The predicted future medical sample events within the forecast time range can allow further predictions relating to the laboratory. For example, it may predict future laboratory operating conditions or requirements, such the expected future processing load, reagent consumption, and personnel requirements of the laboratory.

Herein, the medical sample event data and laboratory data may be collected and processed on an event level (i.e., a level relating to an event occurring with respect to a sample or the laboratory).

In this way, the computer-implemented method is able to predict future operating conditions and medical sample events of a healthcare laboratory. Advantageously, the method collects both medical sample event data and data relating to the laboratory and/or the one or more in-vitro diagnostic laboratory instruments. The medical sample event data may be received from one or more sources external to the laboratory. For example, the data may be received from healthcare providers such as hospitals, general practitioners or other healthcare providers which have requested that the medical samples be processed, as well as from the courier transporting the medical samples. The medical sample event data may be received from one or more sources internal to the laboratory, such as an internal sample tracking system or Laboratory Information System (LIS). Medical sample event data may include data which has been generated inside the healthcare laboratory, generated outside of the healthcare laboratory, or a mixture of the two. Data which has been generated outside of the healthcare laboratory may include, for example, data from an app orchestrating external activities, data entered manually (e.g., into a terminal), data obtained from a local databank (e.g., the LIS) but which originated from outside of the laboratory, and/or may be derived values e.g., calculated, interpolated, and/or experience based.

As such, the method collates data relating to multiple events for each medical sample processed by the healthcare laboratory. This advantageously results in a high volume of available event data, which is converted into a feature set and used as an input for a trained machine learning model. The trained machine learning model is then, thereby, able to accurately forecast future medical sample events. This forecasting is beneficial in managing the laboratory, as it enables staff of the laboratory to process medical samples in a more efficient manner. The trained machine learning model may be a linear Bayesian model; a generalised linear model; a tree-based model (e.g., XGBoost, or light gbm); a recurrent neural network / long short-term memory model; a seasonal autoregressive integrated moving-average with exogeneous regressor model (SARIMAX); or a transformer model.

The laboratory data may comprise data from any of the following data sources: in-vitro diagnostic laboratory instruments, an information system of the healthcare laboratory, and/or a database of laboratory data. In particular, dynamic laboratory data may comprise data from in-vitro diagnostic laboratory instruments, and/or an information system of the healthcare laboratory. Further, in some examples, static laboratory data may comprise data from a database of the healthcare laboratory. The static laboratory data may, in addition or alternatively, comprise data from in-vitro diagnostic laboratory instruments, and/or an information system of the healthcare laboratory.

In-vitro laboratory instruments are used to process the medical samples and send data relating to the medical sample, such as an identification of the sample and the progression of their processing. The augmentation of the medical sample event data with this diagnostic instrument data improves the quality of the feature set such that the trained machine learning model may have an improved ability to predict the times at which future medical samples will undergo processing.

The information system of the healthcare laboratory, which may be a Laboratory Information System (LIS) or hospital information system (HIS), manages information about each medical sample and groups the medical samples by patient. The use of this data in augmentation improves the accuracy of prediction.

The pre-laboratory sample tracking system may take input from a system of a healthcare provider (e.g. a Hospital Information System (HIS), a general practitioner (GP) information system, or a GP entering data directly into the pre-laboratory sample tracking system) that has requested processing of medical samples. Alternatively, or additionally, the pre-laboratory sample tracking system may take input from a system of the courier transporting the medical samples. Use of this data enhances the predictive capability of the trained machine learning model in that the pre-laboratory sample tracking system gives advanced notice that a medical sample has been requested for processing, allowing for the model to forecast when the medical sample will arrive at the laboratory and be processed.

The database of laboratory data may comprise data relating to the layout of the healthcare laboratory and data specific to each in-vitro diagnostic laboratory instrument. The data specific to each diagnostic instrument may be data relating to the type of instrument and the expected throughput of the instrument. The augmentation of the medical sample event data with data from the database of laboratory data allows for the prediction of the trained machine learning model to be specific to the layout and circumstance of a particular laboratory. As such, prediction of future medical sample events may be made with reference to the specific layout and circumstance of the particular laboratory. For example, staff my be allocated to specific areas of the laboratory, and medical samples may be allocated to diagnostic instruments with a higher throughput.

Augmenting the medical sample event data according to the embodiments of the first aspect may include identifying one or more specific medical samples referenced in data obtained from more than one data source and combining the data from each of the data sources which relates to each specific medical sample.

The input for the trained machine learning model according to the embodiments of the first aspect may further include current state data relating to the healthcare laboratory.

This state data may comprise, for example, current utilisation levels and/or statuses for each instrument of the healthcare laboratory, current consumable levels of the laboratory, and current staffing levels and/or current staffing allocations. In this way, the method may be able to recommend improvements or future guidance relating to each of these variables. For example, the method may be able to estimate the number or amount of consumables required to process the predicted volume of medical samples. In another example, the method is able to allocate staff to the processing of medical samples.

Augmenting the medical sample event data according to the embodiments of the first aspect may include identifying incomplete data fields for one or more medical samples.

These incomplete data fields may then be filled in with standard or predicted values. For example, if the incomplete data field is a single event in a chain of events the data associated with the incomplete field may be interpolated. As an example, the data may describe timestamps of when the sample was ordered, received, and processed. Where, for example, the received timestamp is missing, it may be interpolated through using the time of the immediately preceding event plus a mean time difference that can be expected until the missing event. If the algorithm for interpolation is chosen appropriately, null values can also be mitigated. For example, a mean imputation or Amelia II algorithm may be used. To illustrate, assume timestamps for multiple consecutive events are present with an associated timestamp for a single sample. Occasionally, a sample may miss one of the timestamps/events. In that example, there are three events in the series of events: event 1, event 2, and event 3. A sample misses event 2, and so only timestamps for events 1 and 3 are present. Mean imputation can be performed by, for a collection of samples which have the timestamps for event 1 and event 2 populated, calculating the mean time difference between event 1 and event 2 (also calculate the mean time difference between event 1 and even 3, or event 2 and event 3 for other examples), and for a sample with missing event 2 but which has a timestamp for event 1, calculate the possible expected time for event 2 by adding the mean time difference between event 1 and event 2 to the timestamp for event 1.

In a further example, where a class or category of events are missing (e.g., only data pertaining to internal events is accessible, such as sample received and sample processed timestamps) a variation of the prediction algorithm may be implemented which does not require the missing data, or the values for the incomplete data fields may be set to zero. Advantageously, this allows for the creation of a more complete feature set, which results in more accurate predictions.

Both augmented medical sample event data and data relating to the healthcare laboratory according to the embodiments of the first aspect may be used to create the feature set.

Advantageously, the augmentation of the medical sample event data with data relating to the laboratory allows for the prediction of the trained machine learning model to be specific to the layout and circumstance of a particular laboratory.

The laboratory data according to the embodiments of the first aspect may comprise any of:
in-vitro diagnostic laboratory instrument availability data,
in-vitro diagnostic laboratory instrument throughput data,
staff data, and/or
laboratory layout data.

In-vitro diagnostic laboratory instrument availability data is data relating to the availability of the in-vitro diagnostic laboratory instruments of the healthcare laboratory to process medical samples per time bracket. Diagnostic instruments may be unavailable due to the instruments being non-functional or in current or planned use for processing other medical samples. This data is advantageous for prediction in that, in using this data, the model is able to take non-functional and in-use instruments into account in calculating the medical sample throughput of the laboratory. Furthermore, this data allows the model to recommend the use of other operational instruments. In-vitro diagnostic laboratory instrument throughput data is data relating to volume of medical samples which each in-vitro diagnostic laboratory instrument is processes in a given time bracket. Diagnostic instruments of different specifications and types may have different throughputs. Staff data is data relating to the number of staff available for a given time bracket. The use of this data in creating the feature set allows for staff allocation to be recommended, as well as enabling for a more accurate calculation of total laboratory throughput. Laboratory layout data relates to the number of diagnostic instruments of different types in the laboratory, as well as their specific physical layout in the laboratory. As such, prediction of future medical sample events and any related recommendations by the model may be made with reference to the specific of the healthcare laboratory.

The feature set according to the embodiments of the first aspect may include a fitted partial Fourier sum for a given time bracket or for given coefficients of the fit. The Fourier transform allows repeating patterns / seasonality to be straightforwardly derived, and in a computationally lower cost.

The forecast time range according to the embodiments of the first aspect may be defined at least in part by user input.

In this way, a person of the healthcare laboratory may select a forecast time range. A forecast time range may be defined either via a selected time range, or via event identification. For example, a selected time range may specify or two dates and/or times between which the forecast time range is defined. In another example, the system may be instructed that a diagnostic instrument is to become unavailable at a specific time, such that the forecast time range may be selected to precede this event.

Shorter forecasts may be more accurate than long forecasts, however shorter forecasts may provide less time to respond to the prediction of future medical sample events with staff allocation or supply ordering. As such, it is advantageous to allow a person of the laboratory to select the forecast time range, such that more useful predictions may be made given the specific circumstances of the laboratory. Furthermore, this selection may be made via an application of the laboratory.

The computer-implemented method according to the embodiments of the first aspect may further include a step of retraining the trained machine learning model.

Retraining may be trigged either by the expiration of a predetermined retaining time period, for example, the trained machine learning model once per 30 days. Retaining may also be triggered manually, via user input indicating that there has been a change in the setup of the laboratory, and/or via user input indicating that there is an expected future bias in the medical samples ordered. For example, a user may have information that a specific medical centre will stop requesting a specific sample type, and retraining may be used to prompt account for this change.

Moreover, retraining may be triggered by an indication that the trained machine learning model is predicting with an accuracy below a threshold accuracy level. For example, by logging actual medical sample events within the forecast time range and comparing the actual medical sample events within that forecast time range to the predicted future medical sample events for that forecast time range, an accuracy level may be measured. If this accuracy level drops below, for example, a threshold accuracy level of 95% accuracy, that may trigger retraining. Retraining may be advantageous in that it allows the machine learning model to make predictions based on more recent medical sample event data, such that more recent patterns may be exploited to improve the accuracy of prediction. Retraining may also allow for greater volumes of medical sample event data to be used as input to the model as this data progressively become available.

The computer-implemented method of the first aspect may be used to recommend a change to the operation of the healthcare laboratory. For example, the distribution of staff throughout areas of the healthcare laboratory.

The recommendation of a distribution of staff throughout areas of the laboratory may comprise a recommendation on how many staff are required for a given time bracket, which areas of the physical laboratory different numbers of staff should be distributed, as well as a recommendation on the percentage of staff which should be operating in-vitro diagnostic laboratory instruments of different processing types. For example, the laboratory may have a pre-analytic zone, a clinical chemistry zone, and an immunology zone. In this example, the model recommends a distribution of a given number of staff members across these three zones, given the prediction of future processing required. This may be presented as a forecasted volume of samples arriving per area per time unit, or as a distribution of processing requirements per time unit.

A person of the laboratory may be automatically notified when a predicted workload exceeds a threshold workload, such that the person may take action based on further recommendations. This notification may be made via a user interface application. The recommendation of a distribution of staff throughout areas of the laboratory advantageously automates a part of the administrative process of the laboratory, and optimises an otherwise error prone allocation process.

The computer-implemented method of the first aspect may be used to recommend a volume of supplies to be made available.

The volume of supplies to be made available refers to the volume of, for example, reagents or test kits required for processing of the predicted volume of medical samples. This may be calculated primarily based on the throughput of the available in-vitro diagnostic laboratory instruments and the known capacity of reagent kits, wherein this known capacity is stored in a database of the laboratory. This calculation may be made per area of the laboratory, such that each area may be properly supplied. The data may be presented as a forecasted volume of supply use per area per time unit, or as a distribution of supply use per time unit. A recommendation of the volume of supplies to be made available is advantageous in that it allows for supplies to be ordered in advance of sample processing. As such, sample processing can be made more efficient. This supply ordering may be conducted by a manager of the laboratory, or by a computer system. For example, the method may include automatically ordering further supplies where it is determined that the estimated number or amount of supplies required to process the predicted volume of medical samples exceeds an available number or amount of consumables. This automatic ordering is advantageous in that it may be more reliable than ordering conducted by a human, and in that it reduces the administrative burden on staff of the laboratory.

In a second aspect, embodiments of the present invention provide a system for predicting future operating conditions of a healthcare laboratory, the healthcare laboratory containing one or more in-vitro diagnostic laboratory instruments configured to process medical samples, the system comprising one or more processors and memory, wherein the memory contains machine executable instructions which, when executed on the one or more processors, cause the one or more processors to:
receive medical sample event data describing events relating to one or more medical samples to be processed by the laboratory,
augment the medical sample event data with the laboratory data;
create a feature set from the augmented medical sample event data, the feature set including time bracketed data derived from the augmented medical sample event data;
use the feature set as an input for a trained machine learning model, the machine learning model having been trained on historical feature sets to predict a future medical sample events within a forecast time range; and
receive, from the trained machine learning model, a prediction of future medical sample events for the forecast time range as an output.

In a third aspect, embodiments of the present invention provide a computer-implemented method for training a machine learning model to predict future operating conditions of a healthcare laboratory, the healthcare laboratory containing one or more in-vitro diagnostic laboratory instruments configured to process medical samples, the method comprising:
receiving historical medical sample event data describing events relating to one or more medical samples,
augmenting the historical medical sample event data with the laboratory data;
creating a historical feature set from the augmented historical medical sample event data, the historical feature set including time bracketed data derived from the augmented historical medical sample event data; and
using the historical feature set as an input for training a machine learning model to predict a volume of future medical sample events within a forecast time range.

The medical sample event data according to any of the above aspects may describe one or more of:
a medical sample being ordered, and/or
a medical sample arriving at the healthcare laboratory.

The invention may include any one, or any combination insofar as they are compatible, of the optional features set out with reference to the first aspect.

The invention includes the combination of the aspects and preferred features described except where such a combination is clearly impermissible or expressly avoided.

Further aspects of the present invention provide: a computer program comprising code which, when run on a computer, causes the computer to perform the method of the first and/or third aspect; a computer readable medium storing a computer program comprising code which, when run on a computer, causes the computer to perform the method of the first and/or third aspect; and a computer system programmed to perform the method of the first and/or third aspect.

### BRIEF DESCRIPTION OF THE DRAWINGS

**Figure 1** is a flow diagram of a computer-implemented method according to an aspect of the present disclosure.
**Figure 2** is a flow diagram of a training and retraining process according to an aspect of the present disclosure.
**Figure 3** is an example of medical sample event data according to an aspect of the present disclosure.
**Figure 4** is an example of a feature set according to an aspect of the present disclosure.
**Figure 5** is an example of a prediction of future medical sample events for a forecast time range according to an aspect of the present disclosure.
**Figure 6** is an example of a processed prediction of future medical sample events for a forecast time range according to an aspect of the present disclosure.
**Figure 7** is an example of a processed prediction of future medical sample events for recommending a distribution of staff throughout areas of the healthcare laboratory according to an aspect of the present disclosure.
**Figure 8** is an example of a normalised processed prediction of future medical sample events for recommending a distribution of staff throughout areas of the healthcare laboratory according to an aspect of the present disclosure.
**Figure 9** is an example of a normalised processed prediction of future medical sample events for recommending a volume of supplies to be made available according to an aspect of the present disclosure.
**Figure 10** is an example of medical sample event data for use in producing a volume of supplies to be made available according to an aspect of the present disclosure.
**Figure 11** is an example of a prediction of future medical sample events for a forecast time range according to an aspect of the present disclosure.
**Figure 12** is an example of a processed prediction of future medical sample events for recommending a volume of supplies to be made available according to an aspect of the present disclosure.

### DETAILED DESCRIPTION

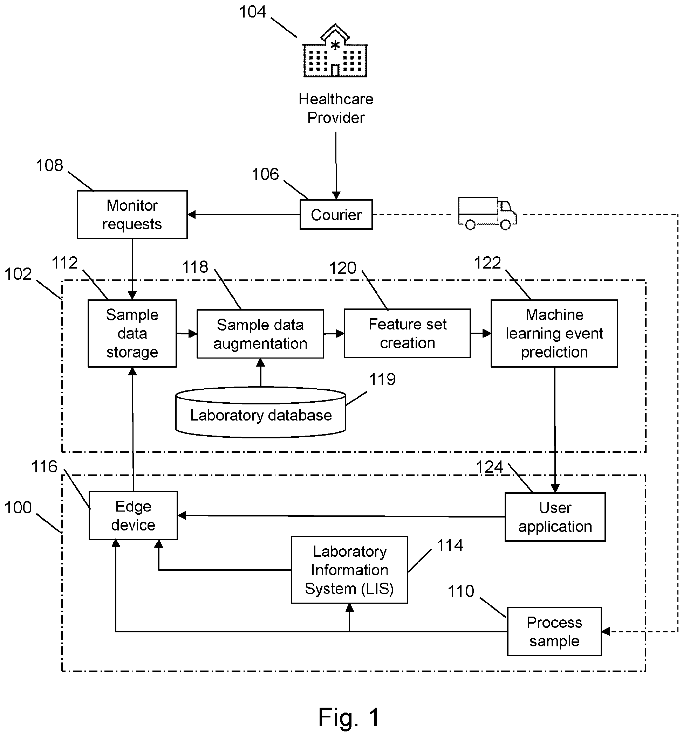

Aspects and embodiments of the present invention will now be discussed with reference to the accompanying figures. Further aspects and embodiments will be apparent to those skilled in the art. Figure 1 shows a flow diagram of a computer-implemented method for predicting future operating conditions of a healthcare laboratory 100 via a cloud system 102 which may comprise one or more processors and memory. The functions described in the cloud system 102 may be implemented, for example, by software running on dedicated hardware components or pooled hardware components. The laboratory contains one or more in-vitro diagnostic laboratory instruments configured to process medical samples according to an aspect of the present disclosure. In a first step, a healthcare provider 104 external to the laboratory requests processing of one or more medical samples. This healthcare provider may be, for example, a health centre, a hospital, a general practitioner, or any other provider of healthcare. This processing request is forwarded to a courier 106, who transports the one or more medical samples for processing to the laboratory 110. All processing requests are also monitored by a request monitoring application 108. The request monitoring application 108 may entirely or in part fulfil the role of the pre-laboratory sample tracking system discussed above, in that it may, upon detecting the processing request, collect medical sample event data relating to the request, such as a timestamp, a sample ID, a reference to the medical test requested on the sample, and the sender of the request, as well as other metainformation. The data is encrypted and sent to a cloud storage system 112 of the cloud system 102.

The laboratory 100 contains an information system 114, for example a Laboratory Information System (LIS), Hospital information System (HIS), or middleware, which manages the data relating to the medical sample order/s for each patient. The information system receives data on arrival and on processing of the medical sample. This data, as well as data directly from the diagnostic instrument(s) processing the medical sample, is sent to an edge device 116. The edge device is a server for securely transmitting data to the cloud storage system 112 and may encrypt the data prior to transmission to the cloud storage system and may be separated from the cloud storage system by a firewall. It uses both standard interfaces (e.g. HL7 and FHIR), and proprietary interfaces (in cases of interface incompatibility) for communication. The information system continuously receives medical sample event data and sends this to the cloud storage system. Each diagnostic instrument also sends data relating to its own availability directly to the cloud storage system.

The medical sample event data is then augmented 118 with laboratory data. In this example, it is data relating to the in-vitro diagnostic laboratory instruments of the laboratory, the information, and the data from the monitoring request application 108. These data sources are merged into a single dataset, which thereby contains more detailed data on the medical samples than it would be possible to obtain from a single source. Further data is pulled from a laboratory database 119 of the cloud system 102, this data comprising the specific layout of the laboratory, specific operating conditions of the laboratory, and data relating to specific in-vitro diagnostic laboratory instruments of the laboratory. As has been discussed previously, the laboratory data may be static and/or dynamic laboratory data. For example, the laboratory data may originate from any of the following data sources: one or more in-vitro diagnostic laboratory instruments (e.g., connected to the LIS 114), an information system of the healthcare laboratory (e.g., LIS 114), and/or a database of laboratory data (119).

This augmented data set is then used to create a feature set 120. Each feature of the feature set is an individually measurable property of the sample events in the forecast time range. The feature set is used as an input to a machine learning model, which is used to predict 122 future medical sample events. This prediction is accessible by personnel in the laboratory via a user application 124. This user application may also be used to set the forecast time range over which the machine learning model makes the prediction.

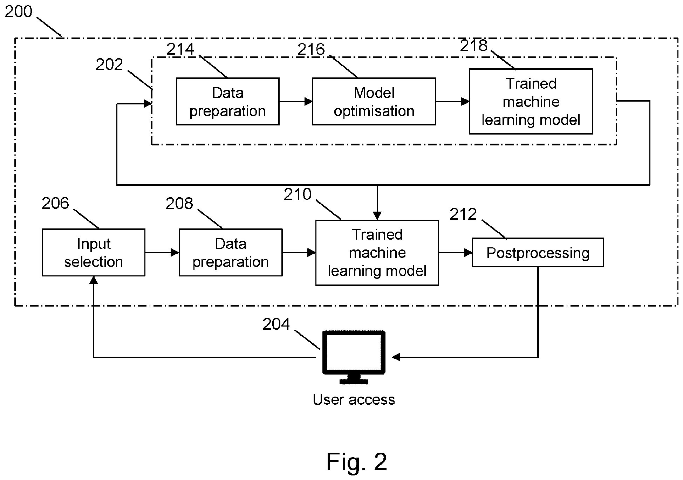

Figure 2 shows a flow diagram of a training and retraining method according to an aspect of the present disclosure. A user inputs a forecast time range 206 into an application 204 within the healthcare laboratory. This input may be via a direct input into a command line interface or graphical user interface, or may be input via a configuration of widgets an application. The input is sent to the cloud 200 and the forecast time range input is then used in the preparation of a feature set 208. The feature set is used as input into a trained machine learning model 210 to produce an output of prediction of future medical sample events for the forecast time range. This output is postprocessed 212 such as to transform the data for use in two applications: staff distribution and supply management.

The trained machine learning module 210 is retrained according to a training method 202. A user defines a historical training time range for training the machine learning model. The duration of this training time range should be selected to be proportional to the intended forecast time range such that, for example, in forecasting a month in advanced, a training time range spanning a year should be used to train the model. This training time range is used to create a feature set 214, which is then optimised for training 216. This optimisation includes the weighting of the feature set to emphasize recent medical sample events. Parameters of the model are optimised based on the training set and are selected based on the validation set. The parameters were, in some examples, optimized with a minimizer trying to minimize a given scoring metric. The minimizer and metric are chosen dependent on the underlying model. An example of a suitable minimizer is the L-BFGS (limited-memory Broyden-Fletcher-Goldfarb-Shanno) algorithm. The validation set comprises a proportion of the original dataset, the original dataset having been split, for example, into a training set, validation set, and test set. In some examples the split is training - 70%, validation - 15%, and test 15%. The models are then trained on the training set, and performance is evaluated on the validation set. The selected model is then tested on the test set, and the performance using this test set is reported back as the metrics for the optimised and selected model.

The resulting trained machine learning model 218 is integrated back into the prediction method, replacing the trained machine learning model 210. Retraining may be trigged either by expiration of a predetermined time period, or by an indication that the trained machine learning model is predicting with an accuracy below a threshold accuracy level.

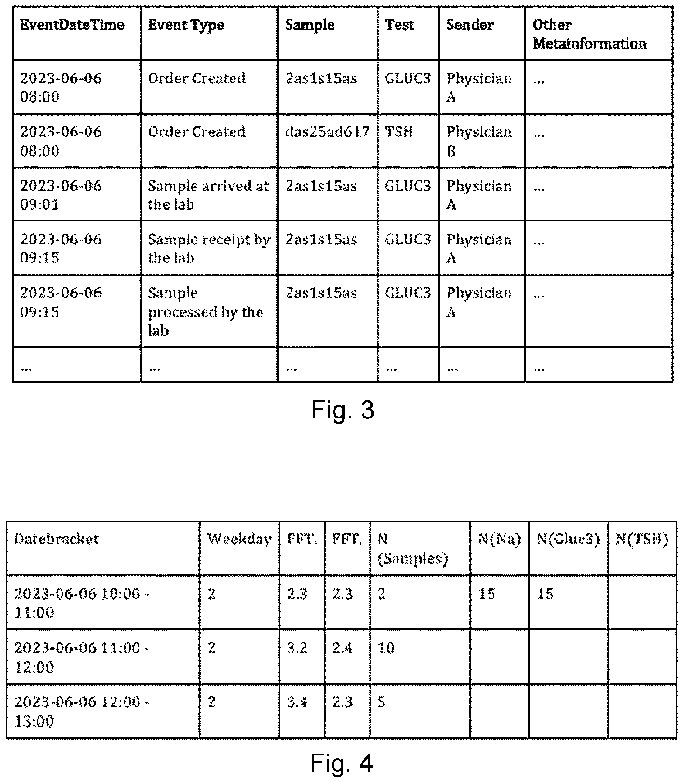

Figure 3 is an example of medical sample event data according to an aspect of the present disclosure. This is an example of raw, un-augmented medical sample event data. Each data point comprises a time stamp describing when the event took place, an event type describing the event that has been monitored, a unique sample ID code for identifying each sample, a test field describing the type of processing that needs to be undergone to process the sample, a sender field describing the healthcare provider which requested the sample, as well as other metainformation relating to the samples that may be specific to the, for example, sender and event type.

Figure 4 is an example of a feature set according to an aspect of the present disclosure. The data of Figure 3 is collated into time brackets. In the example, all sample events from 10:00 to 11:00 on 2023-06-06 are collected and represented in this time bracket. A "weekday", or day of the week, is calculated from the time stamp, "2" corresponding to, for example, a Tuesday. A first and second fitted partial Fourier sum are calculated to account for the seasonality of the data. A number of samples per time bracket and a number of tests of teach type (Na, Gluc3, and TSH in this example) are provided.

Moreover, each time bracket may represent a season quarter of the year, a time of the day, a month, a day of the month, an average number of samples ordered, an average number of tests per sample, an average of tests per sample, a number of a specific test (e.g. Na tests, or GLUC3 tests), sender specific information such as the number of different senders and metrics of a given set of senders (e.g. a number of samples or tests for the set), ordering location specific data such as the number of different senders and metrics of a given set of ordering locations, a receiving location (if the model represents multiple laboratories), the processing area of the laboratory (e.g. ClinChem, immunology, hematology), the number of samples per processing area and/or a total number of staff.

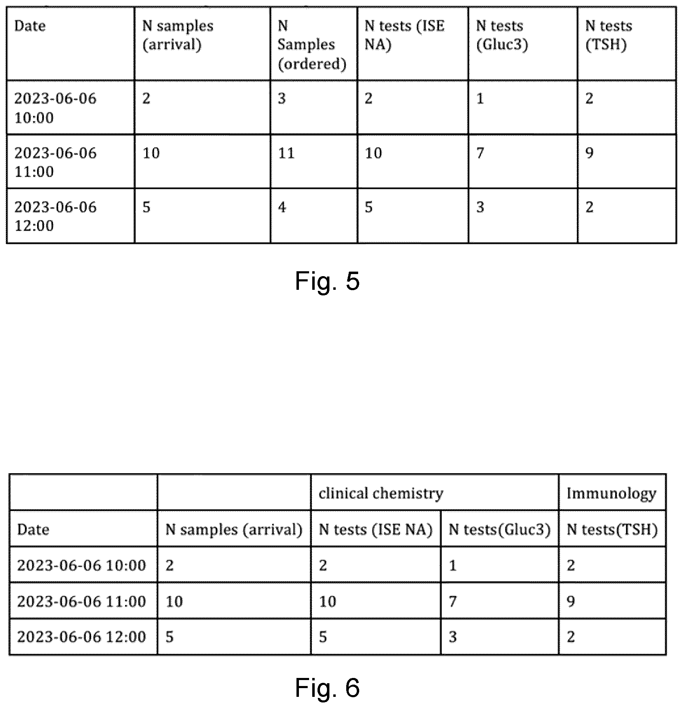

Figure 5 is an example of a prediction of future medical sample events for a forecast time range according to an aspect of the present disclosure. This is a typical output from the trained machine learning model 210. The data comprises the number of medical samples which are predicted to arrive, the number of medical samples which are predicted to be ordered, and the number of each processes which must be done on the in-vitro diagnostic laboratory instruments to process the samples within each 1 hour time bracket.

Figure 6 is an example of a processed prediction of future medical sample events for a forecast time range according to an aspect of the present disclosure. The data has been postprocessed 212 to provide recommendations on the allocation of staff of the healthcare laboratory. This post processing involves the augmenting of prediction data (e.g. that of Figure 5), with laboratory specific data from the laboratory database 119, such as the layout of the laboratory and data specific to each in-vitro diagnostic laboratory instrument. The augmentation of the prediction data with this layout data allows for the prediction of future medical sample events may be made with reference to the specific layout and circumstance of the particular laboratory. Within the data of Figure 6, each processing test is assigned to an area of the laboratory: either clinical chemistry or Immunology. This is advantageous in that the recommendation of the trained machine learning model may allocate staff more optimally to these areas than staff of the laboratory would, thereby improving the efficiency of the laboratory.

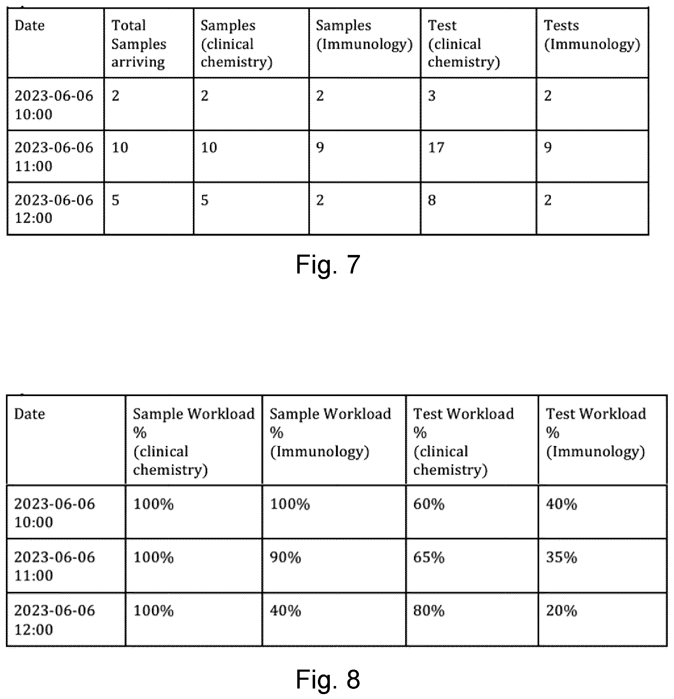

Figure 7 is an example of a processed prediction of future medical sample events for recommending a distribution of staff throughout areas of the healthcare laboratory according to an aspect of the present disclosure. The data of this figure has undergone further postprocessing 212 such as to aggregate the data of Figure 6. Figure 7 provides an aggregate number of medical samples for processing and processing tests for each laboratory area (in this example, clinical chemistry and Immunology). This further postprocessing may make the suggestion data more useful for lab staff while allocating staff to the laboratory.

Figure 8 is an example of a normalised processed prediction of future medical sample events for recommending a distribution of staff throughout areas of the healthcare laboratory according to an aspect of the present disclosure. As such, the data of this figure represents the data of Figure 7 in after the further postprocessing 212 step of normalisation. Each workload is given a percentage of a total workload, such that a corresponding percentage of staff may be allocated to the specific areas of the laboratory (in this example, clinical chemistry and Immunology). This further postprocessing may make the suggestion data more useful for lab staff while allocating staff to the laboratory.

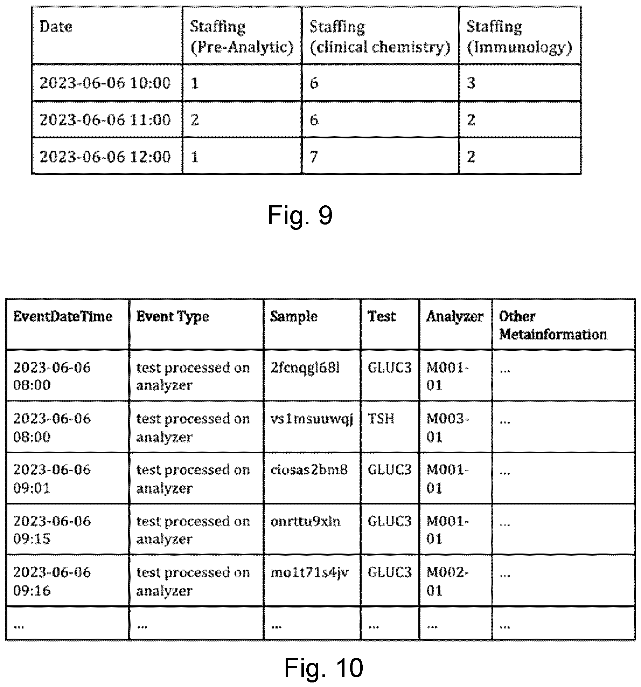

Figure 9 is an example of a normalised processed prediction of future medical sample events for recommending a volume of supplies to be made available according to an aspect of the present disclosure. The data of Figure 9 represents that of Figure 8, with a further processing 212 step of multiplying the normalised prediction distribution with the number of available staff for a specific time bracket. For example, if ten laboratory staff are available, and the clinical chemistry area has a normalised workload of 60% for a specific time bracket, 6 laboratory staff will be assigned to the clinical chemistry area for the specific time bracket. This further postprocessing may make the suggestion data more useful for lab staff while allocating staff to the laboratory.

Figure 10 is an example of medical sample event data for use in producing a volume of supplies to be made available according to an aspect of the present disclosure. This data represents a possible dataset for use in predicting a number of supplies to be used by the laboratory. Notably, this dataset includes event data relating to the processing, or analysis, of each medical sample on a specific in-vitro diagnostic laboratory instrument.. Providing the specific in-vitro diagnostic laboratory instrument used for the processing of each test is advantageous as it allows for the supplies used to be tracked more effectively, thereby enhancing the capability of the trained machine model to predict supply usage.

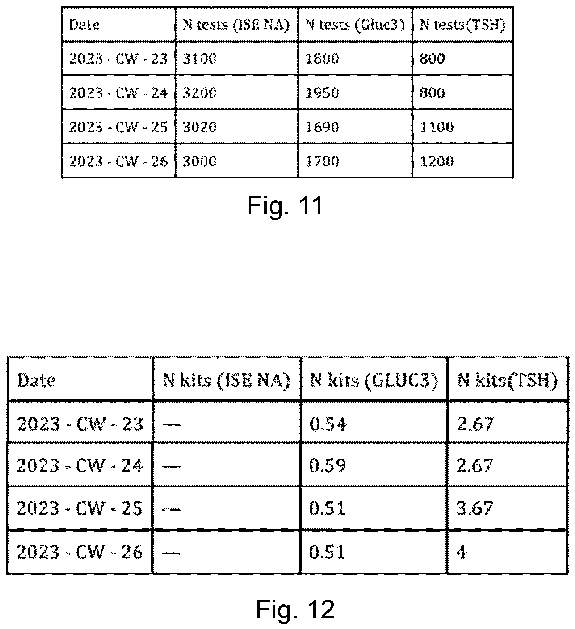

Figure 11 is an example of a prediction of future medical sample events for a forecast time range according to an aspect of the present disclosure. This data represents a postprocessing 212 step of aggregating data over a long time period for use in supply ordering. The medical sample prediction data is aggregated into multiple time brackets, each a week in length. As such, the model is able to predict the total number of processing events of each type per week.

Figure 12 is an example of a processed prediction of future medical sample events for recommending a volume of supplies to be made available according to an aspect of the present disclosure. This data represents a postprocessing 212 step of enhancing the prediction with data from the laboratory database relating to the known capacity of supply kits. This data may be used by laboratory staff to suggest the ordering of a certain number of reagent bottles and/or kits of each type (ISE NA, GLUC3, and TSH in this example). This further postprocessing may make the suggestion data more useful for lab staff while ordering supplies to the laboratory. Furthermore, the user application 124 may notify laboratory staff when supplies of a specific type reach a certain threshold number, or when they reach a number insufficient for the predicted number of processing events of a future time bracket. As such, the laboratory may be made more efficient by reliably having a sufficient number of supplies.

The systems and methods of the above embodiments may be implemented in a computer system (in particular in computer hardware or in computer software) in addition to the structural components and user interactions described.

The term "computer system" includes the hardware, software and data storage devices for embodying a system or carrying out a method according to the above described embodiments. For example, a computer system may comprise a central processing unit (CPU), input means, output means and data storage. The computer system may have a monitor to provide a visual output display. The data storage may comprise RAM, disk drives or other computer readable media. The computer system may include a plurality of computing devices connected by a network and able to communicate with each other over that network.

The methods of the above embodiments may be provided as computer programs or as computer program products or computer readable media carrying a computer program which is arranged, when run on a computer, to perform the method(s) described above.

The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-OMs; electrical storage media such as memory, including RAM, ROM and flash memory; and hybrids and combinations of the above such as magnetic/optical storage media.

While the disclosure has been described in conjunction with the exemplary embodiments described above, many equivalent modifications and variations will be apparent to those skilled in the art when given this disclosure. Accordingly, the exemplary embodiments of the disclosure set forth above are considered to be illustrative and not limiting. Various changes to the described embodiments may be made without departing from the spirit and scope of the disclosure.

In particular, although the methods of the above embodiments have been described as being implemented on the systems of the embodiments described, the methods and systems of the present disclosure need not be implemented in conjunction with each other, but can be implemented on alternative systems or using alternative methods respectively.

The features disclosed in the description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the disclosure in diverse forms thereof.

While the disclosure has been described in conjunction with the exemplary embodiments described above, many equivalent modifications and variations will be apparent to those skilled in the art when given this disclosure. Accordingly, the exemplary embodiments of the disclosure set forth above are considered to be illustrative and not limiting. Various changes to the described embodiments may be made without departing from the spirit and scope of the disclosure.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

**1.** A computer-implemented method for predicting future operating conditions of a healthcare laboratory, the healthcare laboratory containing one or more in-vitro diagnostic laboratory instruments configured to process medical samples, the method comprising:
receiving medical sample event data describing events relating to one or more medical samples to be processed by the laboratory,
augmenting the medical sample event data with laboratory data;
creating a feature set from the augmented medical sample event data, the feature set including time bracketed data derived from the augmented medical sample event data;
using the feature set as an input for a trained machine learning model, the machine learning model having been trained on historical feature sets to predict a future medical sample events within a forecast time range; and
receiving, from the trained machine learning model, a prediction of future medical sample events for the forecast time range as an output.

**2.** The computer-implemented method of claim 1, wherein the medical sample event data is augmented with dynamic laboratory data and static laboratory data.

**3.** The computer-implemented method of claim 1 or claim 2, wherein the medical sample event data includes data from a pre-laboratory sample tracking system, and wherein the laboratory data used to augment the medical sample event data comprises data from any of the following data sources:
in-vitro diagnostic laboratory instruments,
an information system of the healthcare laboratory,
and/or
a database of laboratory data.

**4.** The computer-implemented method of any preceding claim, wherein augmenting the medical sample event data includes identifying one or more specific medical samples referenced in data obtained from more than one data source and combining the data from each of the data sources which relates to each specific medical sample.

**5.** The computer-implemented method of any preceding claim, wherein the input for the trained machine learning model further includes current state data relating to the healthcare laboratory.

**6.** The computer-implemented method of any preceding claim, wherein augmenting the medical sample event data includes identifying incomplete data fields for one or more medical samples.

**7.** The computer-implemented method of any preceding claim, wherein both augmented medical sample event data and data relating to the healthcare laboratory are used to create the feature set.

**8.** The computer-implemented method of claim 6, wherein the laboratory data comprises any of:
in-vitro diagnostic laboratory instrument availability data,
in-vitro diagnostic laboratory instrument throughput data,
staff data, and/or
laboratory layout data.

**8.** The computer-implemented method of any preceding claim, wherein the feature set includes a fitted partial Fourier sum for a given time bracket or for given coefficients of the fit.

**10.** The computer-implemented method of any preceding claim, wherein the forecast time range is defined at least in part by user input.

**11.** Use of the computer-implemented method of any preceding claims for recommending a distribution of staff throughout areas of the healthcare laboratory.

**12.** Use of the computer-implemented method of any of claims 1 to 10 for recommending a volume of supplies to be made available.

**13.** A system for predicting future operating conditions of a healthcare laboratory, the healthcare laboratory containing one or more in-vitro diagnostic laboratory instruments configured to process medical samples, the system comprising one or more processors and memory, wherein the memory contains machine executable instructions which, when executed on the one or more processors, cause the one or more processors to:
receive medical sample event data describing events relating to one or more medical samples to be processed by the laboratory,
augment the medical sample event data with the laboratory data;
create a feature set from the augmented medical sample event data, the feature set including time bracketed data derived from the augmented medical sample event data;
use the feature set as an input for a trained machine learning model, the machine learning model having been trained on historical feature sets to predict a future medical sample events within a forecast time range; and
receive, from the trained machine learning model, a prediction of future medical sample events for the forecast time range as an output.

**14.** A computer-implemented method for training a machine learning model to predict future operating conditions of a healthcare laboratory, the healthcare laboratory containing one or more in-vitro diagnostic laboratory instruments configured to process medical samples, the method comprising:
receiving, historical medical sample event data describing events relating to one or more medical samples,
augmenting the historical medical sample event data with laboratory data;
creating a historical feature set from the augmented historical medical sample event data, the historical feature set including time bracketed data derived from the augmented historical medical sample event data; and
using the historical feature set as an input for training a machine learning model to predict a volume of future medical sample events within a forecast time range.

**15.** The computer implemented method or system of any of the previous claims, wherein the medical sample event data describes one or more of:
a medical sample being ordered, and/or
a medical sample arriving at the healthcare laboratory.
